# EUROPEAN PATENT SPECIFICATION

(11) **EP 2 083 723 B1**
(45) Date of publication and mention of the grant of the patent: **28.12.2011**
(21) Application number: 07854640.5
(22) Date of filing: 14.11.2007
(51) Int. Cl.: A61B 17/72, A61B 17/86

(54) **INTRAMEDULLARY NAIL INCLUDING STABLE LOCKING BOLTS**
MARKNAGEL MIT STABILEN SPERRBOLZEN
CLOU INTRAMÉDULLAIRE COMPRENANT DES BOULONS BLOQUANTS STABLES

(30) Priority: 17.11.2006 US 859531 P
(43) Date of publication of application: 05.08.2009
(73) Proprietor: Synthes GmbH, 4436 Oberdorf (CH)
(72) Inventor: DELL'OCA, Alberto A. Fernadez, Montevideo (UY)
(74) Representative: Lusuardi, Werther
(86) International application number: PCT/US2007/084663
(87) International publication number: WO 2008/064037

(56) References cited:
- DE-A1- 2 246 274
- DE-U1- 9 417 104
- FR-A- 2 799 115
- US-A1- 2004 147 930
- US-B1- 6 355 043
- US-B1- 6 981 976

## Description

The present invention relates to a device for securing an intramedullary implant within a bone according to the preambule of claim 1.

Intramedullary nails for use in the treatment of fractures of bone shafts have included holes close to ends of the nail on both sides of the fracture through which locking bolts were inserted substantially perpendicular to the nail to improve the rotational stability of the fracture and to avoid undesirable bone shortening at the fracture sile. However, movement between the nail and locking bolts may cause difficulties especially where the bone is osteoporotic or where the fracture is close to an end of the bone.

An osteosynthetic aid for tubular bones is known from US 2004/0147930 which includes a locking nail. US 6 355 043 B1 discloses another device according to the preamble of claim 1.

The present invention is directed to a device for securing an intramedullary implant within a bone, comprising a bolt having a first portion extending proximally from a distal end of the bolt and a second portion extending proximally from a proximal end of the first portion to a proximal end of the bolt, wherein a length of the bolt is selected to substantially match a thickness of a portion of bone through which it is to be inserted. A length of the second portion is selected to substantially equal a distance from a point on an outer surface of the bone through which the bolt is to be inserted into the bone to an outer surface of the implant so that, when the bolt is fully inserted into the bone, the distal end of the second portion abuts an outer surface of the implant.

The present invention is further directed to an intramedullary osteosynthetic device for long bones fractures having improved bone holding power. In order to increase the bone holding power of the locked nails, micro-movements between the nail and locking bolts is minimized by applying compression between the nail and the locking bolts. To achieve compression between the nail and locking bolts, a multi-diameter locking bolt is inserted through a threaded nail hole. In addition, if it is desired to reduce the number of different required bolts required in inventory, a two part locking bolt including a front male part and a rear female part may be applied. The male and female parts are preferably strongly locked together to form a unit that functions substantially as a single bolt.

### Surgical technique.

After a nail or other implant has been inserted into the bone (e.g., within the medullary canal), a hole is drilled through the bone aligned with a level of a corresponding hole through the nail. A depth gauge is then used to determine a required total bolt length. The depth gauge is then used to determine a distance between the near bone cortex and the nail to determine the required length of an increased diameter portion of the bolt. Based on the total bolt length required and the required length of the increased diameter portion of the bolt, an appropriately dimensioned bolt is selected. For two part bolts, the required bolt length corresponds to the length of the male part as will be discussed in more detail below while the distance between the nail and the near bone cortex is used to select an appropriately sized female part. The appropriately sized single piece bolt is then inserted through the bone and the nail to lock the nail in the desired position and prevent undesired micro-movements thereof. For a two piece bolt, the selected male part is inserted into the selected female part and these parts are strongly fastened together to form a unit operating substantially as a single bolt -- i.e., to form a two diameter locking bolt. The two diameter locking bolt is inserted through the bone hole and through corresponding nail hole until the larger-diameter-rear-part of the two diameter locking bolt abuts the nail. The bolt is then strongly fastened to the nail to establish compression between the bolt and the nail forcing the combination of the two diameter locking bolt and the nail to operate as a single mechanical unit substantially eliminating micro-movements therebetween.

### Brief Description of the Drawings

Fig. 1 shows a side view of a worm and threaded jacket according to the present invention in a disassembled state;
Fig. 2 shows a side view of the threaded jacket inserted over the worm of Fig. 1;
Fig. 3 shows a cross-sectional view of the worm and threaded jacket of Fig. 1 in the disassembled state;
Fig. 4 shows a cross-sectional view of a threaded jacket inserted over the worm of Fig. 1;
Fig. 5 shows a perspective view of the worm and threaded jacket of Fig. 1;
Fig. 6 shows a side view of a distal end of an intramedullary nail for use in conjunction with the worm and threaded jacket of Fig. 1; and
Fig. 7 shows a cross-sectional view of a bone in which the nail of Fig. 6 and the worm and threaded jacket of Fig. 1 have been inserted.

### Detailed Description

The present invention, which may be further understood with reference to the following description and the appended drawing, relates to devices for treating fractures and, in particular, relates to internal fixation devices for treating fractures. It is noted that, although the exemplary embodiments of the present invention are described below with respect to the treatment of fractures of the femur, the description is not meant to limit the application of the invention to such fractures as the invention may be employed in the treatment of fractures of a number of bones including, for example, the femur, humerus, tibia, ulna, radius, ankle, etc.

To improve the performance of intramedullary nails in osteoporitic bones or in the case of a fracture adjacent to an end of a bone, embodiments of the present invention provide locking screws which substantially eliminate relative movement between an intramedullary nail and the locking screw and which, consequently, minimize relative motion between the intramedullary nail and the bone.

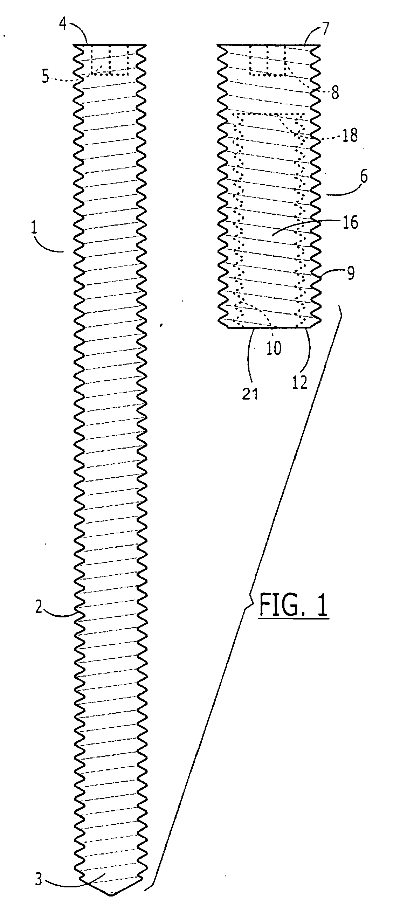

Figs. 1 - 7 show an exemplary embodiment of a device of the present invention comprising an intramedullary nail 11 with a nail hole 13 that may be locked into position within a bone 14 with a two-diameter locking bolt inserted through the nail hole 13. As shown in Figs. 1 - 4, the two diameter locking bolt comprises a separate male part 1 and a female part 6 that may be coupled together to function substantially as a single locking bolt. Fig. 1 shows a two diameter locking bolt according to the invention in a de-coupled state. Male part I is substantially cylindrical and extends from a proximal end 4 to a distal tip 3. The male part 1 has a thread 2 extending substantially helically along its entire length which preferably matches a corresponding thread formed on an internal surface of the nail hole 13 so that the male part 1 may be screwed thereinto. As would be understood by those skilled in the art, the tip 3 may be pointed or otherwise shaped to facilitate inserting the locking bolt into the nail hole 13. A tool receiving feature (e.g., hex recess 5) is formed at the proximal end 4 for use if the male part 1 is inserted first and then, after the male part 1 is in position, the female part 6 is inserted thereover to form the 2 diameter bolt in situ.

The female part 6 is also preferably substantially cylindrical with an outer, bone engaging thread 9 extending substantially helically along its entire length. A proximal end 7 of the female part 6 includes a feature for receiving a tool to be used to turn the female part 6 (e.g., hex recess 8). As would be understood by those skilled in the art, the female part 6 may be turned via this tool receiving feature to couple the female part 6 to the male part 1. A distal end 12 of the female part 6 includes an opening 21 to a lumen 16 including an internal thread 10 (shown in broken lines) which matches the thread 2 of the male part 1 so that the proximal end 4 may be screwed into the lumen 16 until the proximal end 4 abuts a proximal end 18 of the lumen 16. As would be understood by those skilled in the art, the diameter of the lumen 16 is preferably only slightly larger than the diameter of male part 1 so that the male part back 4 of male part 1 may be inserted into the lumen 16 of the female part 6 and the female part 6 may be screwed over the male part 1. As indicated above, the female part 6 may be screwed on male part 1 using a hex key or other tool with a hexagonal bit that fits in the hex recess 7 to drive the female part 6 while male part 1 is held in place, or by simply twisting male part 1 and/or female part 6. It will be understood by those skilled in the art that any type of recess (e.g., slotted, cross-point) may be used instead of the male hex recess 5 and the female hex recess 8 to drive either of the male and female parts 1, 6, respectively.

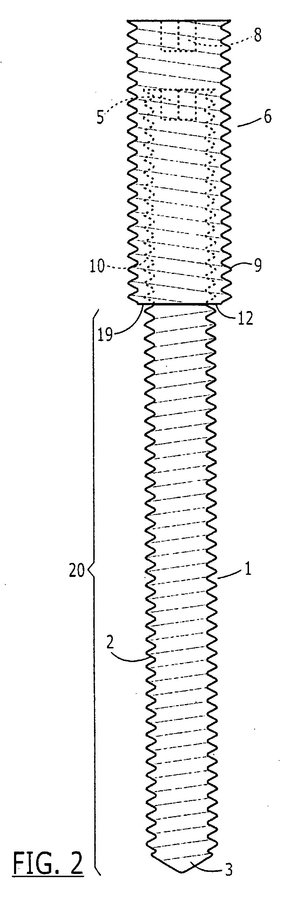

Fig. 2 shows the male part 1 and the female part 6 in an assembled state forming a substantially rigidly bound two-diameter bolt. As shown in broken lines, the female part 6 is coupled to the male part 1 to create a two diameter locking bolt with an annular shoulder 19 formed by the projection of the distal end 12 of the female part 6 radially outward from the portion of the male part 1 adjacent thereto. The female part 6 is preferably shorter than the male part I so that a distal portion 20 of the male part 1 projects distally beyond the end 12 of the female part 6 when the male part 1 and the female part 6 are fully screwed onto one another. Those skilled in the art will understand that male parts 1 of varying length are preferably provided so that a user may select a male part I whose length is substantially equal to a thickness of the bone at the location of the screw hole 13 less a slight clearance for the portion of the female part 6 extending proximally beyond the proximal end 18 of the lumen 16. Then, when a female part 6 is selected having a length substantially corresponding to a distance between the near cortex 15 of the bone 14 and the close side of the nail 11, the female part 6 will screw over the male part 1 until the proximal end 7 of the female part 6 is substantially flush with the outer surface of the bone 14.

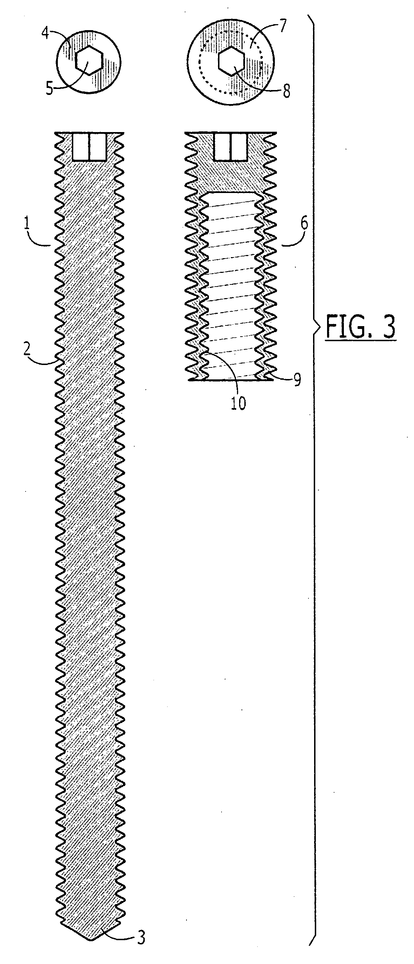

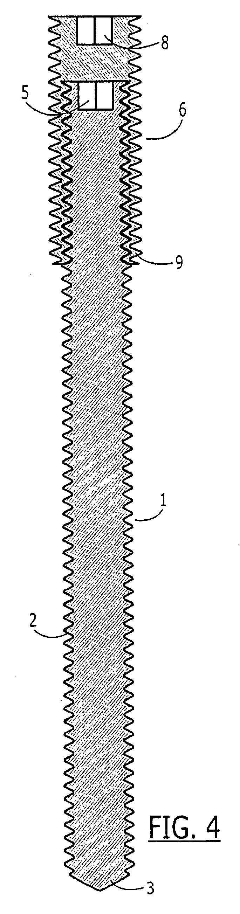

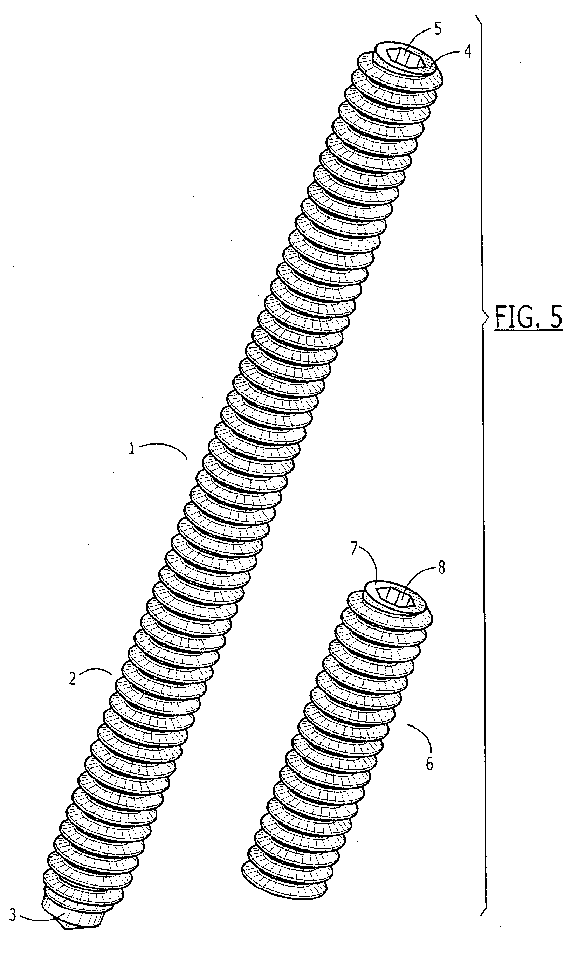

Fig. 3 shows a cross-sectional view as well as a top view of both the male part 1 and the female part 6 in the unassembled state. The top view shows the hex recess 5 at the proximal end 4 of the male part 1 and the hex recess 8 at the proximal end 7 of the female part 6 while the cross section of the female part 6 shows the inner thread 10 of the lumen 16. The diameter of the lumen 16 is shown in broken lines on the top view of female part 6. Fig. 4 shows a cross sectional view of the male part 1 and the female part 6 coupled to one another with the male thread 2 engaging the thread 10 of the lumen 16 with minimal clearance therebetween when the male part 1 and the female part 6 are coupled to one another to minimize or eliminate relative movement between these parts. The pitch of the threading of male part 1 and the female part 6 are also shown in Fig. 5.

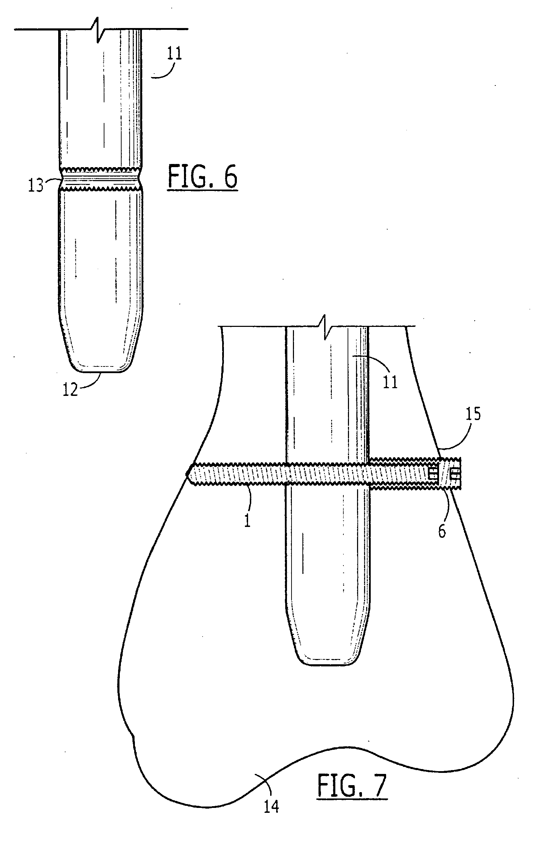

Figs. 6 and 7 show the nail 11 that is to be stabilized by a two-diameter locking bolt according to the present invention. As understood by those skilled in the art, a distal tip 12 of the nail 11 is inserted into a long bone 14 such as, for example, the femur through the medullary canal such that the entire length of the nail 11 is received within the bone 14 and provides stability to a fracture (not shown). Once inserted, a hole is drilled through the bone 14 in alignment with the nail hole 13 so that a hole extends through one side of the bone cortex through the nail 11 (via hole 13) and through the opposite side of the bone cortex on the opposite side of the nail 11 to a position which may, for example, be adjacent to an outer surface of the bone 14. Using a depth gauge, a physician or other medical professional then determines the total length required for the two-diameter bolt to maximize the length of the two-diameter bolt engaging the bone 14 by measuring the distance from the hole in the bone cortex to the far end of the hole. This depth measurement is then used to select a male part 1 with a length which is approximately equal to the total length of the hole minus a thickness of the portion of the female part 6 extending proximally of the proximal end of the lumen 16 as described above. After selection of a male part 1 of the desired length, the depth gauge is used to measure the distance between the nail 13 and the opening into the hole in the near cortex of the bone 14. The user then selects a female part 6 of substantially this second length.

The selected male part 1 and female part 6 are then coupled to one another by inserting the proximal end 4 of the male part 1 into the lumen 16 of the female part 6 so that the thread 2 of the male part 1 engages the inner thread 10 and the two components are rotated relative to one another to securely fasten them together to form a single two-diameter locking bolt. The two-diameter locking bolt is then inserted through the bone hole and the corresponding nail hole 13 by rotating the two-diameter bolt via the hex recess 8 (e.g., using a hex key) until the shoulder 19 abuts the outer surface of the nail 11 surrounding the hole 13. As would be understood by those skilled in the art, at this point, the proximal end 7 of the female part 6 should be substantially flush with the outer surface of the bone 14. At this point, engagement between the nail 11 and the two-diameter locking bolt cause the bolt and the nail 11 to operate as substantially a single mechanical unit minimizing or eliminating micro-movements therebetween.

## Claims

1. A device for securing an intramedullary implant (11) within a bone (14), comprising a bolt (1;6) having a first portion extending proximally from a distal end (3) of the bolt and a second portion extending proximally from a proximal end (4) of the first portion to a proximal end (7) of the bolt (1;6), the length of the bolt being selected to substantially match a thickness of a portion of bone through which it is to be inserted and the length of the second portion being selected to substantially equal the distance from a point on an outer surface of the bone through which the bolt is to be inserted into the bone to an outer surface of the implant so that, when the bolt is fully inserted into the bone, the distal end of the second portion abuts an outer surface of the implant; and wherein the second portion of the bolt (1;6) is provided with a substantially helical exterior thread (9),
**characterized in that**
the bolt (1;6) comprises a first elongate member (1) including a first thread (2) extending along an exterior thereof and - as said second portion - a second elongate member (6) including a lumen (16) extending therein from an opening in a distal end thereof, the lumen (16) including a second thread (10) extending along an inner wall thereof, the first and second threads (2,10) and the diameters of the first and second members (1,6) cooperating so that a proximal portion of the first member (1) is threadably coupleable within the lumen (16), a distal portion of the first member (1) extending distally beyond a distal end of the second member (6) to form the said first portion of the bolt (1;6).

2. The device according to claim 1, wherein an outer diameter of the second portion exceeds an outer diameter of the first portion by a predetermined distance to form a shoulder at the distal end of the second portion.

3. The device according to claim 1 or 2, wherein an outer diameter of the first portion substantially corresponds to an inner diameter of a hole in the implant through which the bolt is to be inserted.

4. The device according to one of the claims 1 to 3, wherein the first and second portions are integrally formed to define a unitary two-diameter bolt.

5. A kit comprising a second member (6) and several first members (1) having a different length of a device according to one of the claims 1 to 3.

6. A kit according to claim 5, comprising several second members (6) having different lengths according to claim 5, of a device according to one of the claims 1 to 3.

7. Assembly comprising a device according to one of the claims 1 - 4 and an intramedullary nail (11) having at least one nail hole (13) adapted to receive the bolt of claim 1.

8. Assembly according to claim 7, wherein said at least one nail hole (13) is provided with an inner thread that matches the first thread (2).

9. Assembly according to claim 7 or 8 for the treatment of bone fractures.

## Patentansprüche

1. Vorrichtung zur Sicherung eines intramedullären Implantats (11) in einem Knochen (14), umfassend:
einen Bolzen (1;6) mit einem ersten Abschnitt, der sich proximal von einem distalen Ende (3) des Bolzens ausdehnt, und einem zweiten Abschnitt, der sich proximal von einem proximalen Ende (4) des ersten Abschnitts bis zu einem proximalen Ende (7) des Bolzens (1;6) ausdehnt, wobei die Länge des Bolzens so gewählt ist, dass sie im Wesentlichen einer Dicke eines Knochenabschnitts angepasst ist, durch welchen der Bolzen eingeführt wird, und die Länge des zweiten Abschnitts so gewählt ist, dass sie im Wesentlichen einer Distanz zwischen einem Punkt auf einer äusseren Oberfläche des Knochens, durch den der Bolzen in den Knochen eingeführt wird, und einer äusseren Oberfläche des Implantates entspricht, so dass, wenn der Bolzen vollständig in den Knochen eingeführt ist, das distale Ende des zweiten Abschnitts an eine äussere Oberfläche des Implantats anstösst; und wobei der zweite Abschnitt des Bolzens (1;6) ein im Wesentlichen helixförmiges Aussengewinde (9) aufweist,
**dadurch gekennzeichnet, dass**
der Bolzen ein erstes longitudinales Teil (1) mit einem ersten Gewinde (2), welches sich entlang einer Aussenfläche davon erstreckt und - als zweiten Abschnitt - ein zweites longitudinales Teil (6) mit einem Lumen (16) umfasst, welches sich in diesem von einer Öffnung in einem distalen Ende davon ausdehnt, wobei das Lumen (16) ein sich entlang einer inneren Wand davon erstreckendes zweites Gewinde (10) umfasst, wobei das erste und zweite Gewinde (2;10) und die Durchmesser des ersten und zweiten Teils (1;6) so zusammenwirken, dass ein proximaler Abschnitt des ersten Teils (1) im Lumen (16) einschraubbar kuppelbar ist, und wobei sich ein distaler Abschnitt des ersten Teils (1) über ein distales Ende des zweiten Teils (6) hinaus erstreckt und den ersten Abschnitt des Bolzens (1;6) bildet.

2. Vorrichtung nach Anspruch 1, **dadurch gekennzeichnet, dass** ein Aussendurchmesser des zweiten Abschnitts einen Aussendurchmesser des ersten Abschnitts um einen vorgegebenen Abstand überschreitet, um eine Schulter am distalen Ende des zweiten Abschnittes zu bilden.

3. Vorrichtung nach Anspruch 1 oder 2, **dadurch gekennzeichnet, dass** ein Aussendurchmesser des ersten Teils im Wesentlichen einem Innendurchmesser eines Lochs im Implantat entspricht, durch welches der Bolzen eingeführt wird.

4. Vorrichtung nach einem der Ansprüche 1 bis 3, **dadurch gekennzeichnet, dass** der erste und zweite Abschnitt integral ausgebildet sind, um einen einheitlichen Bolzen mit zwei Durchmessern zu definieren.

5. Kit umfassend ein zweites Teil (6) und mehrere erste Teile (1) mit einer unterschiedlichen Länge einer Vorrichtung nach einem der Ansprüche 1 bis 3.

6. Kit nach Anspruch 5 umfassend mehrere zweite Teile (6) mit unterschiedlichen Längen einer Vorrichtung nach einem der Ansprüche 1 bis 3.

7. System umfassend eine Vorrichtung nach einem der Ansprüche 1 - 4 und einen Marknagel (11) mit mindestens einem zur Aufnahme des Bolzens nach Anspruch 1 angepassten Nagelloch (13).

8. System nach Anspruch 7, **dadurch gekennzeichnet, dass** das mindestens eine Nagelloch (13) ein Innengewinde aufweist, welches mit dem ersten Gewinde (2) zusammenpasst.

9. System nach Anspruch 7 oder 8 zur Behandlung von Knochenfrakturen.

## Revendications

1. Dispositif permettant de fixer un implant intramédullaire (11) dans un os (14), comprenant un boulon (1 ; 6), ayant une première partie qui s'étend proximalement depuis une extrémité distale (3) du boulon et une seconde partie qui s'étend proximalement depuis une extrémité proximale de la première partie vers une extrémité proximale (7) du boulon (1 ; 6), la longueur du boulon étant choisie pour correspondre sensiblement à une épaisseur d'une partie d'os dans lequel il doit être inséré, et la longueur de la deuxième partie étant choisie pour être sensiblement égale à la distance allant d'un point sur une surface externe de l'os par lequel le boulon doit être inséré dans l'os à une surface externe de l'implant, de sorte que, lorsque le boulon est pleinement inséré dans l'os, l'extrémité distale de la deuxième partie vient en butée contre la surface externe de l'implant, et dans lequel la deuxième partie du boulon (1 ; 6) est pourvue d'un filetage extérieur sensiblement hélicoïdal,
**caractérisé en ce que**
le boulon (1 ; 6) comprend un premier élément allongé (1) incluant un premier filetage (2) s'étendant le long d'une partie extérieure de celle-ci et - en tant que ladite deuxième partie - un deuxième élément allongé (6) incluant une lumière (16) s'y étendant depuis une ouverture dans une extrémité distale de celle-ci, la lumière (16) incluant un deuxième filetage (10) s'étendant le long d'une paroi intérieure de celui-ci, les premier et deuxième filetages (2, 10) et les diamètres des premier et deuxième éléments (1, 6) coopérant de sorte qu'une partie proximale du premier élément (1) est couplée par filetage avec la lumière (16), une partie distale du premier élément (1) s'étendant distalement au-delà d'une partie distale du deuxième élément (6) pour former ladite première partie du boulon (1 ; 6).

2. Dispositif selon la revendication 1, dans lequel un diamètre externe de la deuxième partie excède un diamètre externe de la première partie d'une distance prédéterminée de façon à former un épaulement au niveau de l'extrémité distale de la deuxième partie.

3. Dispositif selon la revendication 1 ou 2, dans lequel un diamètre externe de la première partie correspond sensiblement à un diamètre interne d'un trou dans l'implant par lequel le boulon doit être inséré.

4. Kit selon l'une quelconque des revendications 1 à 3, dans lequel les première et deuxième parties sont formées solidairement afin de définir un boulon unitaire à deux diamètres.

5. Kit comprenant un deuxième élément (6) et plusieurs premiers éléments (1) ayant une longueur différente d'un dispositif selon l'une quelconque des revendications 1 à 3.

6. Kit selon la revendication 5, comprenant plusieurs seconds éléments (6) ayant des longueurs différentes d'un dispositif selon l'une quelconque des revendications 1 à 3.

7. Ensemble comprenant un dispositif selon l'une quelconque des revendications 1 à 4 et un clou intramédullaire (11) comportant au moins un trou de clou (13) adapté pour recevoir le boulon de la revendication 1.

8. Ensemble selon la revendication 7, dans lequel ledit au moins un trou de clou (13) est pourvu d'un filetage interne qui correspond au premier filetage (2).

9. Ensemble selon la revendication 7 ou 8, pour le traitement des fractures osseuses.
